# EUROPEAN PATENT APPLICATION

(11) **EP 2 682 496 A1**
(43) Date of publication of application: **08.01.2014**
(21) Application number: 12004937.4
(22) Date of filing: 03.07.2012
(51) Int. Cl.: C23C 8/06, C23C 8/80, C23C 8/02, C23C 8/10, A61L 27/04, A61L 31/14, C21D 3/02, C22F 1/02

(54) **Method of modification of surface structure of metal body**

(71) Applicant: Linde Aktiengesellschaft, 80331 Munich (DE)
(72) Inventor: Foret, Pierre, 80796 München (DE); Hulstein, Eric, 81927 München (DE)
(74) Representative: Gellner, Bernd

(57) **Abstract**

Method for the modification of the surface structure of a metal body, wherein in a first stage, a layer containing at least one non-metallic substance is created on the surface of the metal body, and in a second stage the layer is at least partly removed from the surface of the metal body, wherein in the first stage, the metal body is exposed to a first heat treatment in a first defined gas atmosphere, and in the second stage the metal body is exposed to a heat treatment in a second defined gas atmosphere,
characterized in that, wherein following the first heat treatment and/or the second heat treatment, a cooling of the metal body performed, the cooling being effected at a rate of between 250°C and 5°C, especially between 220°C and 10°C, or between 220°C and 13°C to 14°C per minute.

## Description

The invention relates to a method for the modification of the surface structure of a metal body.

Stainless austenitic steels after their manufacture frequently have a smooth metal surface without structure and can therefore be frequently wetted only with difficulty. This wetting characteristic has a major influence on the adhesion and durability of paints and coatings, i.e. the application of durable coatings on such surfaces proves difficult.

In medical technology, biocompatible materials such as for example titanium are used for producing implants from these. With such implants, too, a surface that is too smooth also results in problems that are difficult to solve. Among these are for example poor contact between the implant and the human tissue in which the implant is implanted.

In the paper " Porous Metal Tubular Support for Solid Oxide Fuel Cell Design", Electrochemical and Solid-State Letters Volume 9, No. 9, Pages A427 to A429, June 2006 , a method for the manufacture of a porous nickel tube is described. To this end, the nickel tube is initially oxidized and subsequently reduced in a hydrogen atmosphere.

The formation of nickel pores at certain temperatures and after certain times is assumed to be due to special relations between the thermal stability of the NiO and the diffusion rates of nickel and oxygen atoms in nickel metal and in NiO.

Contrary to the oxidation of nickel, in Fe or Co containing metals, not only one oxide but more oxides of the type MO, M2O3 and M3O4, with M = Fe or Co, with different thermal stabilities are formed depending on the oxidation temperature. In addition, in Fe-based or Co-based alloys, oxides formed by alloying ellements such as Cr, Mo, Mn, and Si may be formed which make the picture even more complex. During reduction, the diffusion of Fe or Co and of the alloying elements in the matrix and in the oxides creates an extremely complicated picture.

The same arguments as for Fe- or Co-based alloys also apply for other metal alloys with additions of Cr, Fe, Cu, Co, Mo, Mn and Si, and for Ta- and Ti-based alloys.

A method according to the preamble of claim 1 in known from EP 1 935 508 A1.

The object of the present invention therefore is to provide a method for increasing the surface porosity of a metal body.

This object is solved by a method for the modification of the surface structure of a metal body comprising the features of claim 1.

The method of the invention comprises two stages: In a first method stage comprising a first heat treatment a surface layer is created on the metal body which has at least one nonmetallic element or a compound containing one nonmetallic element. In the first method stage, preferably carbon, oxygen, nitrogen, sulphur or phosphorus are installed in the surface layer as nonmetallic elements. The creation of this surface layer need not take place in one step, but can also be carried out in several steps.

After this, a nonmetallic element or a compound containing these nonmetallic elements can be removed again partly or wholly from the surface layer in a second method stage comprising a second heat treatment. In this way, vacancies remain in the surface layer which bring with them a porosity of the surface. This second method stage of the removal of the nonmetallic elements from the surface layer can also take place in one or several steps.

By suitably selecting the composition of the heat treatment atmosphere and corresponding selection of the process parameters such as for example pressure or temperature it is possible in this way to bring about reactions between the components of the heat treatment atmosphere and the surface of the metal body in a controlled manner and to produce a defined surface layer. Be it noted in this connection that parameters such as treatment time, dewpoint or gas composition can also be selected in order to optimise the process.

The method of the invention is **characterized in that** a rapid cooling of the metal body is performed following the first heat treatment and/or the second heat treatment. By subjecting a metal body to such a rapid cooling, significantly smaller pores in the surface of the metal body as compared to prior art solutions can be created. A comparison to prior art solutions, as for example described in EP 1 935 508 A1, shows that the method of the invention can create pores of an average size of about 200 to 800 nm, as compared to about 1 to 50 microns (µ), in the prior art.

The invention utilizes a cooling rate of between 250°C and 5°C, especially between 220°C and 10°C, or preferably between 220°C and 13°C to 14°C per minute.

Thus, according to the invention, pores on a nanoscale can be provided in a simple cost effective way.

Such a surface structure has numerous advantages, for example in connection with heat transfer to boiling liquids, in which the metal body is immersed. Pool boiling effects are advantageously influenced by such a surface structure comprising nanoscale pores. The pores provided by the invention lead to an enhancement of the formation of vapor bubbles and vapor columns by means of which the heat transfer between a metal body modified according to the invention and a liquid can be significantly improved. The invention can be performed consecutively a number of times, e.g. comprising the first stage, followed by the second stage, followed by a further first stage and a further second stage etc.

Advantageously there is no need for a final (surface structure modifying) treatment of the surface after the above mentioned second method stage. In particular, the surface layer is not subjected to an etching process or a similar process.

Advantageous embodiments are the subject matter of the dependent claims.

Preferably, the cooling following the first heat treatment and/or the second heat treatment is effected at a rate of between 176°C and 22°C, especially between 100°C and 50°C per minute.

Preferably, in the first stage the layer is created in the first defined gas atmosphere at a temperature of between 800°C and 1300°C, especially between 1000°C and 1200°C or between 1050°C and 1150°C. These temperatures allow a particularly effective surface creation and partial removal.

Preferably, the first defined gas atmosphere is an oxidizing atmosphere, and/or the second gas atmosphere is a reducing atmosphere. The terms oxidation and reduction are to be understood in the broadest of terms, i.e. loss of electrons and gain of electrons respectively.

During the oxidation step an oxide layer forms on the metal surface in the known manner. In a second step the oxidized metal surface is now exposed to a reducing atmosphere. During this treatment at least a part of the existing oxides is removed through reduction, wherein corresponding pores remain in the surface.

By carrying out oxidation followed by reduction of the metal surface a porous surface structure that can be easily wetted is created.

Preferably, the oxidizing atmosphere comprises oxygen and/or water and/or carbon dioxide, and preferably inert gas, and/or the reducing atmosphere comprises hydrogen and/or carbon monoxide.

It is considered especially advantageous that the oxidizing atmosphere has an oxygen content of at least 50%, preferably 75% or 90% and/or the reducing atmosphere has a hydrogen content of at least 75%, preferably at least 90% or 99% or 100%. However, oxidizing atmospheres with an oxygen content of as little as 0 to 1% (i.e. smaller than 1 %) can be utilized.

Preferentially air is used as oxidizing agent. Depending on the metal to be oxidized and the desired shape of the pore structure however it may also be favourable to carry out the oxidation with the help of air enriched with oxygen or technically pure oxygen as oxidizing agent. Preferably an atmosphere with an oxygen content of at least 50%, especially preferably at least 90% of oxygen is used here. If applicable, the temperature of the oxidation treatment has to be suitably adjusted in this case. It has also been shown also shown that other oxidizing agents such as for example moist air, steam, carbon dioxide or mixtures of nitrogen and oxygen as well as other non-inert gases are suitable to bring about the oxidation according to the invention.

In an embodiment of the invention the oxidation of the surface can likewise develop or be created as a side effect during another heat treatment or transformation step. During hot rolling, for instance, the surface of the metal is already oxidized so that additional separate oxidation need not necessarily be performed.

The reduction of the oxide layer is preferentially carried out in a hydrogen atmosphere. Here it has proved itself to employ a reducing atmosphere with hydrogen content of at least 75%, preferably at least 90%, especially preferably at least 98% or 99% or 100% of hydrogen. Instead or in addition to hydrogen, an atmosphere containing CO can also be used for reduction.

Excellent results are achieved in case the heat treatment in the first stage is effected for a period of time between 2 and 200 minutes, preferably between 2 and 120 minutes, or between 2 and 80 minutes, or between 2 and 20 minutes or preferably between 4 and 7 minutes and/or the heat treatment in the second stage is performed over a period of time between 2 and 200 minutes, preferably between 2 and 120 minutes, or between 2 and 80 minutes, or between 2 and 20 minutes or preferably between 4 and 7 minutes. Preferably, the heat treatment in the first and second stage can be effected over the same or similar periods of time.

It is advantageous that the first stage and/or the second stage comprises several individual method steps, wherein in each method step at least one non-metallic substance, preferably C, O, N, S or P, is deposited in or on the surface layer and/or at least partly removed from the surface layer.

In this preferred embodiment the first method stage and/or the second method stage consist of several method steps, wherein in each method step at least one nonmetallic substance is deposited in the surface layer and/or removed from the surface layer. The metal body can for example be initially modified in a treatment atmosphere so that a nonmetallic element is installed in the surface of said body, for example the metal surface is oxidized through controlled reaction with an atmosphere containing oxygen. After this, the metal body is again subjected to the same treatment in order to bring about an additional installation of the nonmetallic element in the surface structure of the metal body, i.e. in the mentioned example the metal surface would be exposed to an additional oxidation reaction.

The same applies to the second method stage: the removal of the nonmetallic components from the surface layer can likewise take place in several steps. Dividing the two method stages of the installation or the removal of the nonmetallic components into several steps is more preferably practical when a larger amount of these nonmetallic substances is to be installed in or removed from this surface, but prolonged or more intensive treatment of the metal body has disadvantageous effects on the metal properties. Through the mentioned division of a method stage into several steps performed in succession a more careful treatment of the metal body is achieved.

The division of a method stage into several steps is also advantageous if a plurality of different substances is to be installed in or removed from the surface structure of the metal body. To this end, the reaction conditions are optimized in a first step such that preferably a certain nonmetallic substance reacts with the surface of the metal body. In a second step the reaction conditions are modified so that another substance is integrated in the surface layer. Additional steps for the controlled modification of the surface can follow. Of course this does not only apply to the installation of the nonmetallic elements in the surface but also to their removal in a second method stage.

Preferentially the two-stage process according to the invention, i.e. the sequence of the first and the second method stage, is carried out repeatedly. This means a porous surface layer is initially created through the deposition and the at least partial removal again of the mentioned substances. The surface of the metal body pretreated in this way is then preferentially subjected one more time to the method according to the invention under changed method conditions. As a result, a surface with different size pores can be produced. For example a coarsely structured surface, which has inner surfaces with a fine structure, can be created through the appropriate choice of the method parameters. Such surfaces with coarse and fine porosities are for example of advantage for the manufacture of heat exchanger surfaces or catalytic converter surfaces.

The method is especially advantageous if the metal body comprises or is constituted of a metal alloy comprising at least one of the metals Fe, Cu, Co, Cr, Ti, Ta, Mo, Mn and Si as primary component or as addition.

Especially advantageous is to perform the method according to the invention on a metal alloy containing at least one of the following Fe, Cu, Co, Cr, Ti Ta, Mo, Mn, Si. Preferably it can be performed on stainless steel or stainless austenitic steels.

A preferred application of the inventive method is with respect to devices for medical or pharmaceutical purposes, preferably implants. The invention is preferably utilized for modifying the surface structure of devices for medical or pharmaceutical purposes. For example medical implants e.g. tooth implants, stents, doctor's instruments, catheters, prostheses or artificial joints or materials of which such implants and prostheses are manufactured, are modified according to the invention.

The surface porosity improves the contact between the implant and the human or animal tissues or bones. On the other hand, the implants or prostheses, but also doctor's instruments, are frequently provided with coatings, for example an hydroxyapatite coating. Through the use of the method according to invention clearly improved adhesion of such layers is achieved.

More preferably in the area of medical technology and surgery, but also in other technical areas, can the pores formed according to the invention be utilized in order to deposit active substances, isotopes, radioactive substances for combating cancer or pharmaceuticals in said pores which are to be given off to the environment or introduced in the surrounding tissue.

It is especially advantageous that, utilizing the enhanced/increased porosity of the surface, an active substance, preferably an active substance that is active in a medical or a pharmaceutical manner, is deposited in the pores as provided by the invention.

Finally, a preferred application of the method according to the invention is to use steel alloyed with chromium or a stainless steel as metal alloy.

The type and shape of the surface porosity created depends on the method parameters during the first method stage and during the second method stage. In order to obtain a pore structure which is optimally adapted to the set requirements the parameters, more preferably temperature and duration, have to be suitably selected in both the method stages.

These parameters depend on the type of the metal or the alloy whose surface is to be modified, on the substance that is to be installed in or removed from this surface layer, on the type of the oxidizing and/or reduction agent employed and on the desired pore structure, for example their size. If the surface modification through heat treatment of the metal body according to the invention is performed in a gas atmosphere, the temperature of the atmosphere and the respective exposure times are therefore adjusted to the surface to be treated as a function of the type of the metal or the alloy, as a function of the type of the oxidation and reduction agent employed and/or as a function of the desired pore structure.

It has been shown that with the treatment of Co-Cr alloys with a relatively high carbon content according to the invention a part of the carbon atoms present in the alloy is utilized in the second method stage for the reduction of the nonmetallic substances. The observed characteristic of carbon as reduction agent can for example be deliberately employed in that carbon is added to the hydrogen or CO atmosphere used in the second method stage.

In a preferred embodiment of the invention the oxidation is carried out in air and the subsequent reduction in an atmosphere of pure hydrogen.

The objective of the method according to the invention is to create a porous metal surface. To this end, non-metals are initially deposited in the surface of the metal body during the first method stage, for example an oxidation step, and, during the second method stage, for example during a reduction step, are removed again so that the desired pores remain. Both these method stages are advantageously carried out in immediate succession. More preferably it is of advantage if the surface is not exposed to any other heat treatment process between the two method stages, more preferably between an oxidation and a reduction step.

The method is used to advantage in order to treat a metal alloy containing at least one of the following Fe, Cu, Co, Cr, Ti Ta, Mo, Mn, Si according to the invention. Preferably, it can be performed on stainless steel or stainless austenitic steels. Here, either the entire body or only the surface of the body to be treated is successively exposed to the two method stages, i.e. for example initially to an oxidizing and subsequently to a reducing atmosphere.

Another preferred area of application of the method according to the invention is the treatment of metal surfaces in order to improve their adhesion characteristics for subsequent painting or coating.

Additional areas of application of the invention are the modification of the surface structure of heat exchangers in order to improve the heat transfer and the flow conditions along the heat exchanger surfaces. The modified surfaces according to the invention can also bring advantages in catalytic converters and batteries, as well as for example water boilers and water heaters.

It has also been shown that the optical properties of surfaces, for example the absorption capacity, can be influenced in a controlled manner through the invention. A potential area of application for this are solar collectors.

At present, surfaces requiring a defined structure or porosity are frequently produced through powder coating or siritering-on of powder. The present invention constitutes a cost-effective possibility of superseding these relatively expensive methods.

Finally it is also possible with thin metal bodies to not only modify their surface but to produce a body according to the invention which is porous throughout. Such porous metal bodies for example can be employed as filters.

It has been shown that during the two method stages of the creation or the removal of the nonmetallic components increased diffusion of alloying elements into the metal surface occurs. Advantageously the method according to the invention is therefore utilized to alloy the metal surface in a controlled manner. Especially preferably a few micrometer thick surface layer is created on the metal body in this manner, in which the Cr or Mo-content compared with the remainder of the surface layer is increased. It was for example discovered that the Cr-content in this outermost layer can be increased by 5 to 15%.

This controlled enrichment of elements in the outermost surface layer has more preferably great advantages in such applications where the corrosion resistance of the surface is of great importance, for example in order to protect medical implants from acids produced by the body.

A further preferred area of application of the invention is the increase of the surface hardness, more preferably of micromechanical or electronic components. With the suitable selection of the process parameters a surface layer with particularly small grain size is formed. This is attributed to the fact that the metal atoms which remain after the removal of the nonmetallic atoms in the second method stage compose themselves into new grains. If, in the process, very many new small grains per unit area are formed, this results in a high surface hardness.

The invention has numerous advantages compared with the prior art. With the method according to the invention the surface porosity can be customized. Depth and size of the pores can be set through suitable selection of the method parameters in the oxidation and in the reduction steps. More preferably stainless austenitic steels, Co-Cr-alloys, titanium and tantalum materials, which frequently have a smooth surface structure, can be prepared according to the invention so that subsequent coatings last better and durably.

The invention will now be further described referring to the following example.

### Example 1:

A steel body made of stainless steel was oxidized in an oxygen atmosphere at 1100°C for 6 minutes. Following oxidation, the body was cooled at a rate of 75°C/min, until room temperature was reached.

Subsequently, the steel body was reduced in a 100% hydrogen atmosphere at 1100°C, for 7 minutes. Following reduction, the steel body was again cooled at a rate of 75°C/min, until room temperature was reached.

Using SEM-imaging of the surface of the steel body after performing these steps, the porosity of the surface was clearly visible. It could be shown that the average pore diameter is between 200 nm and 800 nm.

It is possible to perform the method according to the invention in a belt furnace. However, the process is not limited to continuous furnaces. Batch furnaces may also be used.

## Claims

1. Method for the modification of the surface structure of a metal body, wherein in a first stage, a layer containing at least one non-metallic substance is created on the surface of the metal body, and in a second stage the layer is at least partly removed from the surface of the metal body, wherein in the first stage, the metal body is exposed to a first heat treatment in a first defined gas atmosphere, and in the second stage the metal body is exposed to a heat treatment in a second defined gas atmosphere,
**characterized in that**,
following the first heat treatment and/or the second heat treatment, a cooling of the metal body is performed, the cooling being effected at a rate of between 250°C and 5°C, especially between 220°C and 10°C, or between 220°C and 13°C to 14°C per minute.

2. Method of claim 1, **characterized in that** the cooling following the first heat treatment and/or the second heat treatment is effected at a rate of between 176°C and 22°C, especially between 100°C and 50°C per minute.

3. Method according to anyone of the preceding claims, **characterized in that** in the first stage the layer is created in the first defined gas atmosphere at a temperature of between 800°C and 1.300°C, especially between 1.000°C and 1.200°C or between 1.050°C and 1.150°C.

4. Method according to any one of the preceding claims, **characterized in that** in the second stage the layer is at least partly removed in the second defined gas atmosphere at a temperature of between 900°C and 1.400°C, preferably between 1.200°C and 1.300°C.

5. Method according to any one of the preceding claims, **characterized in that** the first defined gas atmosphere is an oxidizing atmosphere, and/or the second defined gas atmosphere is a reducing atmosphere.

6. Method according to claim 5, **characterized in that** the oxidizing atmosphere comprises oxygen and/or water vapor and/or carbon dioxide, and preferably inert gas, and/or the reducing atmosphere comprises hydrogen and/or carbon monoxide and/ or non inert gases.

7. Method according to claim 5 or 6, **characterized in that** the oxidizing atmosphere has an oxygen content of up to or under 1%, or of at least 50 %, preferably 75 % or 90 %, and/or the reducing atmosphere has a hydrogen content of at least 75 %, preferably at least 90 %, 99 % or 100%.

8. Method according to anyone of the preceding claims, **characterized in that** the heat treatment in the first step is effected for a period of time between 2 and 200 minutes, preferably between 2 and 120 minutes, or 2 and 80 minutes or between 2 and 20 or between 4 and 7 minutes, and/or the second step performed over a period of time between 2 and 200 minutes, preferably between 2 and 120 minutes, or 2 and 80 minutes or between 2 and 20 or between 4 and 7 minutes.

9. Method according to any one of the preceding claims, **characterized in that** the first stage and/or the second stage comprises several method steps, wherein in each method step at least one non-metallic substance, preferably C, O, N, S or P, is deposited in or on the surface layer and/or removed from the surface layer.

10. Method according to any one of the preceding claims, **characterized in that** the sequence of the first and second stages is carried out repeatedly.

11. Method according to any one of the preceding claims, **characterized in that** it is performed on a metal alloy containing at least one of the following Fe, Cu, Co, Cr, Ti Ta, Mo, Mn, Si, especially on stainless steel or stainless austenitic steels.

12. Method according to any one of the preceding claims, **characterized in that** it is performed on devices for medical or pharmaceutical purposes, preferably of implants.

13. Method according to any one of the preceding claims, **characterized in that** an active substance, preferably an active substance that is active in a medical or pharmaceutical manner, is deposited in the pores on the surface structure.

14. Method according to any one of the preceding claims, **characterized in that** the metal alloy is steel alloyed with chromium or a stainless steel.
